# EUROPEAN PATENT SPECIFICATION

(11) **EP 4 192 407 B1**
(45) Date of publication and mention of the grant of the patent: **26.08.2026**
(21) Application number: 21749312.1
(22) Date of filing: 26.07.2021
(51) Int. Cl.: A61F 13/00

(54) **APPARATUS FOR CONTROLLING LEAKAGE OF A REDUCE PRESSURE THERAPY SYSTEM**
VORRICHTUNG ZUR STEUERUNG DER LECKAGE EINES UNTERDRUCKTHERAPIESYSTEMS
APPAREIL PERMETTANT DE RÉGULER UNE FUITE AU NIVEAU D'UN SYSTÈME DE THÉRAPIE À PRESSION RÉDUITE

(30) Priority: 06.08.2020 US 202063062218 P
(43) Date of publication of application: 14.06.2023
(73) Proprietor: KCI Manufacturing Unlimited Company, Athlone, Co. Westmeath, N37XF22 (IE)
(72) Inventor: ENRIQUEZ, Alec, Dublin Road Athlone, Co. Westmeath, N37XF22 (IE); MALDONADO, Pedro, Dublin Road Athlone, Co. Westmeath, N37XF22 (IE); MARCHAND, Brenda, Dublin Road Athlone, Co. Westmeath, N37XF22 (IE); KHARKAR, Prathamesh Madhav, Dublin Road Athlone, Co. Westmeath, N37XF22 (IE)
(74) Representative: Simmons & Simmons
(86) International application number: PCT/IB2021/056739
(87) International publication number: WO 2022/029557

(56) References cited:
- WO-A1-2019/036169
- KR-A- 20110 009 718
- US-A1- 2019 054 218

## Description

### TECHNICAL FIELD

The invention set forth in the appended claims relates generally to tissue treatment systems and more particularly, but without limitation, to controlling the leakage of a negative pressure wound therapy system.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," and "vacuum-assisted closure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

WO 2019/036169 A1 concerns apparatuses and methods for removing fluid from a wound utilizing controlled air-flow. US 2019/054218 A1 concerns a manually activated negative pressure therapy system with integrated audible feedback.

While the clinical benefits of negative-pressure therapy are widely known, the cost and complexity of negative-pressure therapy can be a limiting factor in its application, and the development and operation of negative-pressure systems, components, and processes continues to present significant challenges to manufacturers, healthcare providers, and patients.

### BRIEF SUMMARY

New and useful systems for maintaining negative pressure in low and high leak conditions in a negative-pressure therapy environment are set forth in the appended claims. Illustrative embodiments are also provided to enable a person skilled in the art to make and use the claimed subject matter.

The reduced pressure provided by a reduced pressure treatment system to a tissue site such as, for example, an incision or a wound, may need to be properly controlled to increase the effectiveness of the reduced pressure treatment. The reduced pressure treatment system may include a pump for providing reduced pressure, a wound dressing disposed adjacent the wound, and a drape that covers both to provide a sealed environment for providing the reduced pressure treatment from the pump to the sealed environment. However, leaks may occur in the dressing and other components of the reduced pressure treatment system such as, for example, leaks between the drape and the tissue site, i.e., system leakage. The system leakage may be a high system leakage or a low system leakage depending on the flow rate of fluids within the system especially adjacent the dressing. In a low system leakage system, it may be necessary to monitor and control the flow rate to ensure that the flow rate does not drop below a minimum value, i.e., a minimum flow rate, to maintain effective treatment of a wound at the tissue site. If the flow rate drops below the minimum flow rate, the system may be configured to increase the flow rate by inducing leakage of air from the external environment into the therapeutic environment of the dressing to maintain effective treatment of the wound.

The invention provides a system according to claim 1.

In some example embodiments, the flow rate sensor may comprise a first pressure sensor having a first input for sensing the pump pressure (PP) with a first output for providing a signal indicative of the pump pressure (PP), and a second pressure sensor having a second input for sensing the wound pressure (WP) with a second output for providing a signal indicative of the wound pressure (WP). The controller may be electrically coupled to the first output of the first pump and the second output of the second pump and may be configured further to determine the flow rate (FR) based on the difference between pump pressure (PP) and the wound pressure (WP).

Also disclosed herein is a method for stimulating healing of a wound at a tissue site may comprise positioning a porous pad at the tissue site and covering the porous pad with a drape to form a therapeutic environment separated from the external environment for maintaining a wound pressure (WP) at the tissue site. The method may further comprise applying negative pressure to the porous pad using a pump to generate a pump pressure (PP) for applying negative pressure to the tissue site. The method may comprise further determining a flow rate (FR) of fluids between the pump and the porous pad indicative of a leak and providing a flow-rate signal, and then comparing the flow rate (FR) to a minimum flow rate (MinFR). If the flow rate (FR) measured is less than the minimum flow rate (MinFR), the method further comprises generating a low-leak signal and increasing the flow rate (FR) in response to the occurrence of a low-leak signal by increasing the leakage between the pump and the porous pad.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

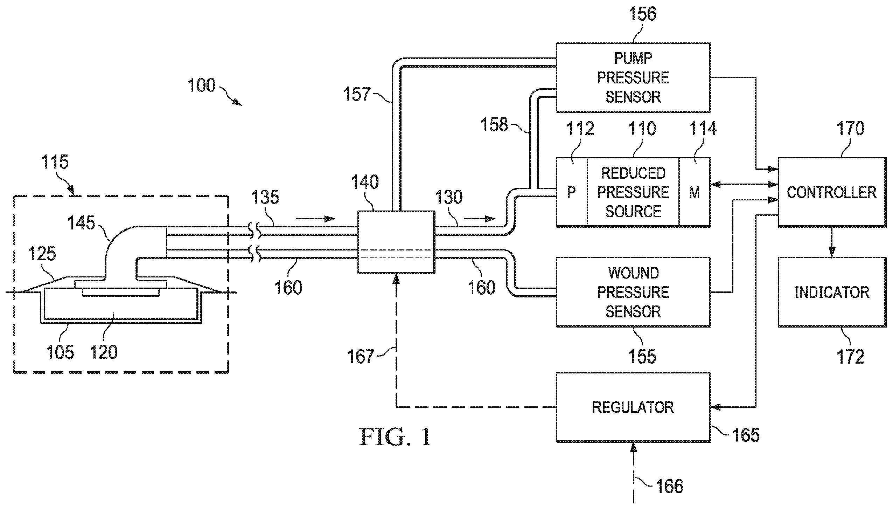

Figure 1 is a functional block diagram of an embodiment of one example of a reduced-pressure therapy system including a controller coupled to a pump motor and a pump that can provide hybrid control of pressure being provided to a tissue site and control of leakage of the reduced type pressure therapy system in accordance with this specification;

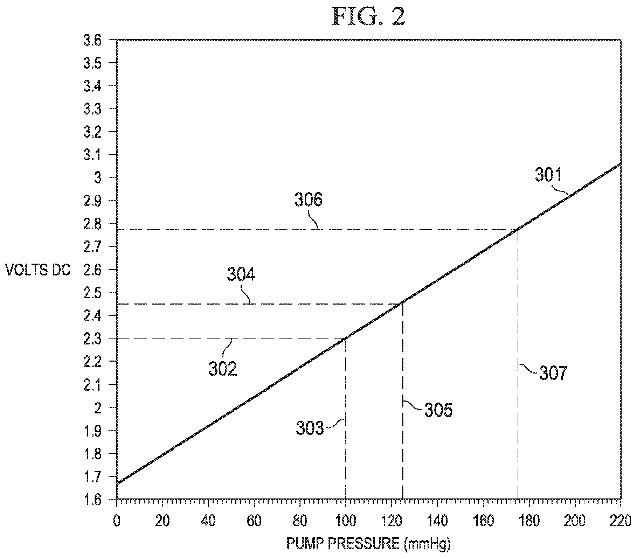

Figure 2 is a graph illustrating stall voltage characteristics for a pump motor that may be used in the reduced pressure therapy system of Figure 1 wherein the x-axis represents the vacuum pressure loading for the pump motor and the y-access represents the stall voltage;

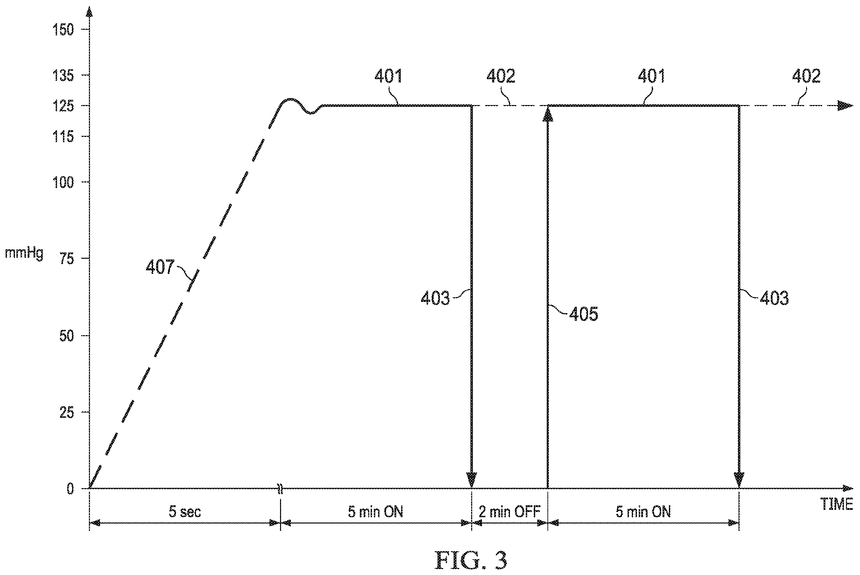

Figure 3 is a graph illustrating pressure control of a motor-drive system in accordance with an illustrative embodiment of the example embodiment wherein the x-axis represents time in minutes (min) and/or seconds(sec) and the y-axis represents pressure generated by a pump in Torr (mmHg) that varies with time in a continuous control mode and an intermittent mode that may be used in the reduced pressure therapy system of Figure 1;

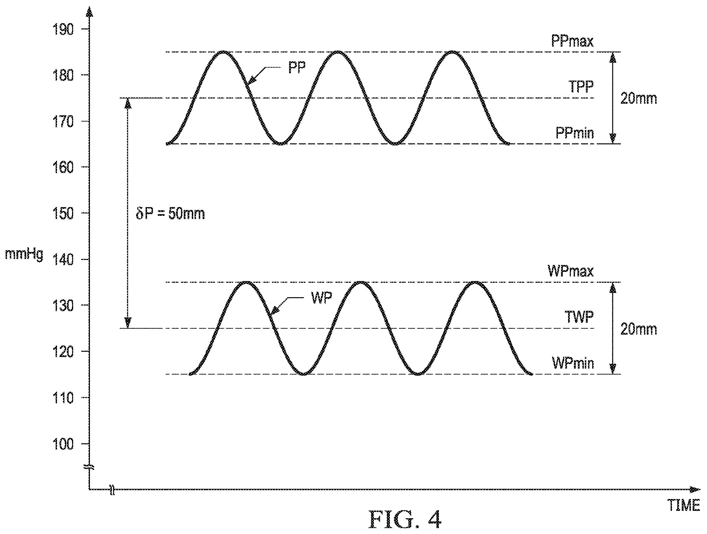

Figure 4 is a graph illustrating pressure control of a motor-drive system in accordance with an illustrative embodiment of the example embodiment wherein the x-axis represents time in minutes (min) and/or seconds(sec) and the y-axis represents pressure generated by a pump in Torr (mmHg) that compares a manipulated variable, such as a tissue site or wound pressure (WP) at a tissue site, with a control variable, such as a pump pressure (PP), for use with a PID controller and/or a bang-bang controller;

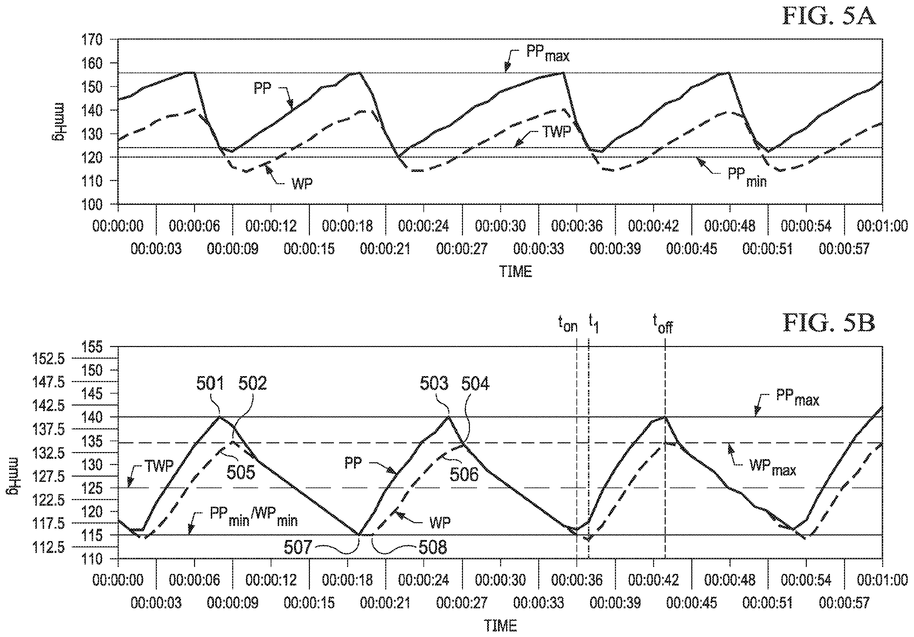

Figures 5A and 5B are graphs illustrating pressure control for a bang-bang controller in accordance with an illustrative embodiment of the example embodiment wherein the x-axis represents time in seconds(sec) and the y-axis represents pressure generated by a pump in Torr (mmHg) that varies with time in an continuous control mode and wherein the pressure control of the bang-bang controller is subjected to a larger head pressure created by the reduced-pressure's therapy system of Figure 1 as shown in Figure 5A compared to the smaller head pressure shown in Figure 5B;

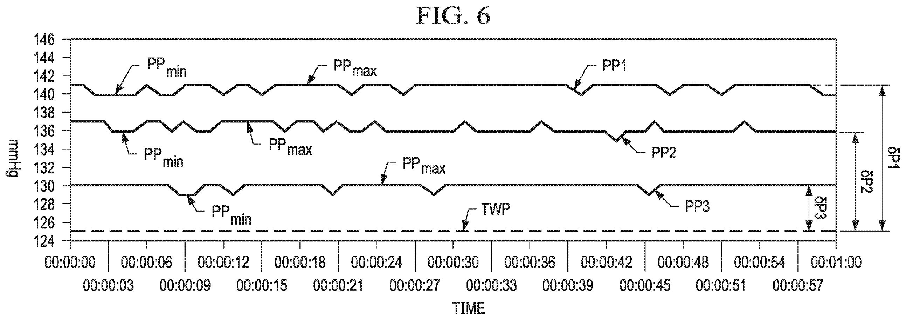

Figure 6 is a graph illustrating pressure control for a PID controller in accordance with an illustrative embodiment of the example embodiment wherein the x-axis represents time in seconds(sec) and the y-axis represents pressure generated by a pump in Torr (mmHg) that varies with time in an continuous control mode and wherein the horizontal time scale is substantially the same as the horizontal timescale shown in Figure 5B for comparing pressure control with that of the bang-bang controller;

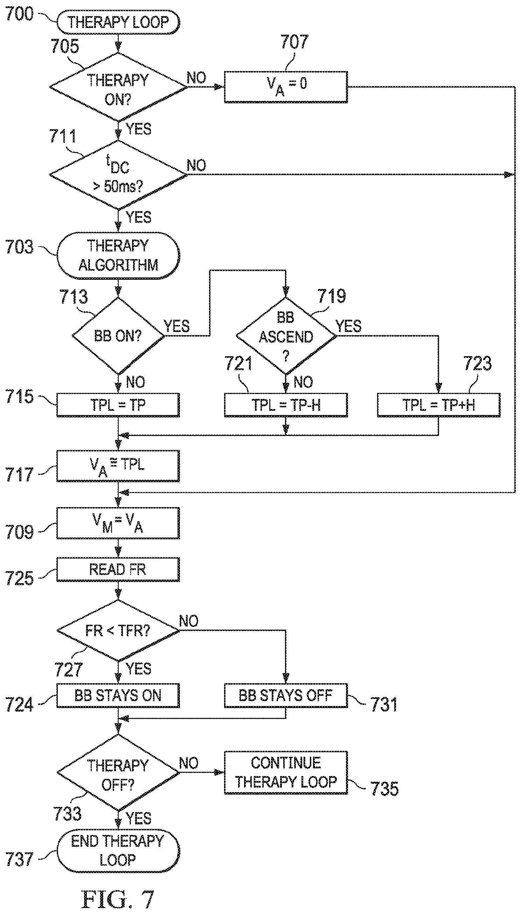

Figure 7 is a flowchart illustrating a process or therapy loop for controlling reduced pressure at a tissue site that may be stored on the controller of Figure 1 including a therapy algorithm for selecting the appropriate pump pressure control for controlling reduced pressure at a tissue site in accordance with an illustrative embodiment of the example embodiment; and

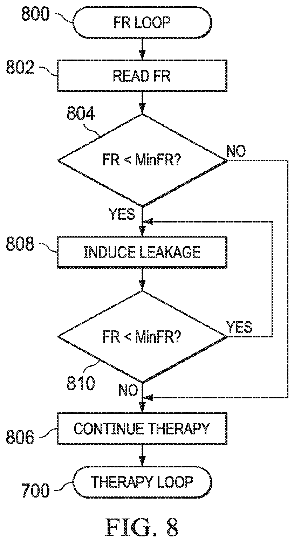

Figure 8 is a flow chart illustrating a process or flow-rate loop for controlling gaseous leakage from the external environment into the therapeutic environment of the reduced pressure therapy system in accordance with an illustrative embodiment of the example embodiment.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

Figure 1 is a simplified functional block diagram of an example embodiment of a reduced-pressure therapy system 100 that can provide negative-pressure therapy in accordance with this specification. More specifically, the therapy system 100 may be used for controlling which pump pressure control is utilized to provide the appropriate amount of reduced pressure to tissue site 105. Tissue site 105 may be the bodily tissue of any human, animal, or other organism, including bone tissue, adipose tissue, muscle tissue, dermal tissue, vascular tissue, connective tissue, cartilage, tendons, ligaments, or any other tissue. While tissue site 105 may include a wound, diseased tissue, or defective tissue, the tissue site may further include healthy tissue that is not wounded, diseased, or defective. The application of reduced pressure to tissue site 105 may be used to promote the drainage of exudate and other liquids from tissue site 105, as well as promote the growth of additional tissue. In the case in which tissue site 105 is a wound site, the growth of granulation tissue and removal of exudates and bacteria promotes healing of the wound. The application of reduced pressure to non-wounded or nondefective tissue, including healthy tissue, may be used to promote the growth of tissue that may be harvested and transplanted to another tissue location.

The reduced pressure applied to the tissue site 105 may be provided by a reduced pressure source 110. Reduced pressure source 110 may be any type of manually, mechanically, or electrically operated pump. Non-limiting examples of reduced pressure source 110 include devices that are driven by stored energy, and which are capable of producing a reduced pressure. Examples of these stored energy, reduced pressure sources include, without limitation, pumps driven by piezoelectric energy, spring energy, solar energy, kinetic energy, energy stored in capacitors, combustion, and energy developed by Sterling or similar cycles. Still other devices and processes that may be used or included in reduced pressure source 110 include syringes, lead screws, ratchets, clockwork-driven devices, pendulum-driven devices, manual generators, osmotic processes, thermal heating processes, and processes in which vacuum pressures are generated by condensation. In another embodiment, reduced pressure source 110 may include a pump 112 wherein the pump 112 provides negative or reduced pressure, i.e., a pump pressure (PP), to the tissue site 105 that may be driven by a motor 114 electrically coupled to a controller 170 which is also a component of the reduced-pressure therapy system 100, also referred to as a system controller. The motor 114 may be a direct-current motor powered by a DC power supply such as, for example, a battery (not shown). Preferably, the pump 112 uses low amounts of power and is capable of operating for an extended period of time on a single charge of the battery such as, for example, a diaphragm pump.

In one example embodiment, the reduced pressure source 110 comprises a DC motor 114 powered by a battery, i.e., the applied power. The applied power can be varied to control the speed of the motor by varying either the current or the voltage applied to the motor, i.e., the "applied voltage" (V_{A}). The applied voltage (V_{A}) may be varied, for example, by modulating the voltage with a square wave and varying the duty cycle of the square wave to control the speed of the DC motor 114. The reduced pressure source 110 also comprises a pump 112 that provides a reduced pressure or vacuum to the tissue site 105. Consequently, the pump 112 represents the load on the DC motor 114 so that when the therapy requires that the reduced pressure at the tissue site 105 needs to be increased, the applied voltage (V_{A}) provided to the DC motor 114 is increased to achieve the targeted reduced pressure at the tissue site 105. One skilled in the art knows that the DC motor 114 will not run or turn the pump until the applied voltage (V_{A}) is sufficient to overcome the inertia or load of the pump 112, which in this case may be a diaphragm pump.

Referring more specifically to Figure 2, a graph 301 illustrating the voltages for the pump motor 114 necessary to start the pump 112 is shown wherein the X-axis represents the pump pressure (PP) loading the DC pump motor and the Y-axis represents the applied voltage (V_{A}). For example, the controller 170 may need to apply at least 2.3V to the DC motor 114 before it will turn the pump 112 when loaded at a pressure of 100 mmHg as indicated by the dashed lines 302, 303. Applying any less than 2.3V to the DC motor 114 would yield insufficient power for the motor to turn the pump, i.e., the loaded motor would remain stopped or "stalled" so that the motor is unable to turn the pump. Hence, the 2.3V value is often referred to in the industry as the "stall voltage" that would be calculated for a DC motor under a load of 100 mmHg of pressure, i.e., the "stall pressure." Correspondingly, the controller 170 may need to apply a larger voltage of at least 2.45V 304 to the DC motor before it will turn the pump when loaded at a greater pressure of 125 mmHg. Applying any less than the stall voltage of 2.45V to the DC motor would not be sufficient to cause the DC motor to turn the pump under a stall pressure of 125 mmHg. Variations in the stall voltage are proportional to the variations in the pressure load on the motor, i.e., the greater the pressure load is on the motor, the greater the stall voltage needed to overcome the pressure load.

The specific stall voltage for a specific DC motor used to drive a diaphragm pump can typically be determined by one skilled in the art from the specifications available for the DC motor. The diaphragm pump and DC motor may be and integrated device such as, for example, a *Thomas Model No. 30130002* series 4.5V diaphragm pump for which such information is readily available. (Thomas; thomas.de@gardnerdenver.com) Referring again to Figure 2, the graph 301 illustrating the stall voltage for the pump motor, the Y-axis represents the stall voltages calculated for this Thomas motor based on the specifications presently available at the Thomas website referred to above. The examples provided in the paragraph above include voltages and pressures that are exemplary only. The graph 301 simply illustrates that one skilled in the art can calculate the various stall voltages for a DC motor based on specifications typically available for that motor. Those working with miniature diaphragm pumps that are driven by a DC motor, such as the Thomas DC motor, often refer to the stall voltage as the "stall power", i.e., the product of the stall voltage and the rated current of the specific DC motor.

Data from pump specifications is typically limited to the relation of maximum flow to vacuum pressure at maximum pump voltage (e.g., 4.5V for Thomas pump identified above). Positive pressures are specified in mbar units (mmHg of positive pressure = 0.7500616827042 * mbars) and vacuum pressures are specified in terms of percent vacuum. For example, if 100% maximum vacuum is specified at 760mmHg, 40% maximum vacuum would be equal to 304 mmHg of vacuum (= 0.4 * 760mmHg). In this example, the 304 mmHg of vacuum pressure would be the theoretical maximum vacuum pressure that we could attain if the pump was run at 4.5V and allowed to run until it the DC motor stalls. The graph 301 in Figure 2 was generated based on the motor specifications and the stall voltages observed that were needed to drive this pump.

The equation for calculating the stall voltage for this particular pump is as follows: *Stall Voltage* = *1.638V + (0.006515V*/*mmHg * XmmHg),* where X is the current vacuum pressure. Therefore, at 50 mmHg of vacuum, the stall voltage equals 1.96V (1.638 + (0.006515 * 50)); at 125 mmHg of vacuum, the stall voltage equals 2.45V (1.638 + (0.006515 * 125)) as indicated by the dashed lines 304, 305; and at 175 mmHg of vacuum, the stall voltage equals 2.78V (1.638 + (0.006515 * 175)) as indicated by the dashed lines 306, 307. Again, the higher the vacuum pressure, the higher the applied voltage that is needed to start the pump. Otherwise, the pump stalls and will not move until the necessary stall will voltage is applied. When the pump stalls, the DC motor simply overheats which can damage the DC motor and reduce battery life.

Referring back to Figure 1, the reduced pressure source 110 may provide reduced pressure to the tissue site 105 via a dressing 115. Dressing 115 may include a tissue interface such as, for example, a manifold 120 which may be placed adjacent to or in contact with the tissue site 105. Manifold 120 may be a biocompatible, porous material that is capable of being placed in contact with tissue site 105 and distributing reduced pressure to the tissue site 105. Manifold 120 may be made from foam, gauze, felted mat, or any other material suited to a particular biological application. Manifold 120 may include a plurality of flow channels or pathways to facilitate distribution of reduced pressure or fluids to or from tissue site 105.

In one embodiment, manifold 120 is a porous foam and includes a plurality of interconnected cells or pores that act as flow channels. The porous foam may be a polyurethane, open-cell, reticulated foam such as GranuFoam manufactured by Kinetic Concepts, Inc. of San Antonio, Texas. If an open-cell foam is used, the porosity may vary, but is preferably about 400 to 600 microns. The flow channels allow fluid communication throughout the portion of manifold 120 having open cells. The cells and flow channels may be uniform in shape and size, or may include patterned or random variations in shape and size. Variations in shape and size of the cells of manifold result in variations in the flow channels, and such characteristics may be used to alter the flow characteristics of fluid through manifold 120. The manifold 120 may further include portions that include "closed cells." These closed-cells portions of manifold 120 contain a plurality of cells, the majority of which are not fluidly connected to adjacent cells. Closed-cell portions may be selectively disposed in manifold 120 to prevent transmission of fluids through perimeter surfaces of manifold 120.

Manifold 120 may also be constructed from bioresorbable materials that do not have to be removed from a patient's body following use of reduced pressure treatment system 100. Suitable bioresorbable materials may include, without limitation, a polymeric blend of polylactic acid (PLA) and polyglycolic acid (PGA). The polymeric blend may also include without limitation polycarbonates, polyfumarates, and capralactones. Manifold 120 may further serve as a scaffold for new cell-growth, or a scaffold material may be used in conjunction with manifold 120 to promote cell-growth. A scaffold is a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials. In one example, the scaffold material has a high void-friction (i.e., a high content of air).

The dressing 115 may also include sealing member 125 also referred to as a drape or cover. Manifold 120 may be secured to tissue site 105 using sealing member 125. Sealing member 125 may be a cover that is used to secure manifold 120 at tissue site 105. While sealing member 125 may be impermeable or semi-permeable, in one example sealing member 125 is capable of maintaining a reduced pressure at tissue site 105 after installation of the sealing member 125 over manifold 120. Sealing member 125 may be a flexible drape or film made from a silicone based compound, acrylic, hydrogel or hydrogel-foaming material, or any other biocompatible material that includes the impermeability or permeability characteristics desired for tissue site 105. Sealing member 125 may be formed of a hydrophobic material to prevent moisture absorption by the sealing member 125. In one embodiment, sealing member 125 is configured to provide a sealed connection with the tissue surrounding manifold 120 and tissue site 105. The sealed connection may be provided by an adhesive (not shown) positioned along a perimeter of sealing member 125 or on any portion of sealing member 125 to secure sealing member 125 to the manifold 120 or the undamaged epidermis peripheral to a tissue site, i.e., the peritissue. The adhesive may be pre-positioned on sealing member 125 or may be sprayed or otherwise applied to sealing member 125 immediately prior to installing sealing member 125.

In general, components of the therapy system 100 may be coupled directly or indirectly. Components may be fluidly coupled to each other to provide a path for transferring fluids (i.e., liquid and/or gas) between the components. In some embodiments, for example, components may be fluidly coupled through a tube. A "tube," as used herein, broadly refers to a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may additionally or alternatively be coupled by virtue of physical proximity, being integral to a single structure, or being formed from the same piece of material. Coupling may also include mechanical, thermal, electrical, or chemical coupling (such as a chemical bond) in some contexts

The reduced pressure generated by reduced pressure source 110 may be applied to tissue site 105 through source tube 130 and delivery tube 135. Source tube 130 and delivery tube 135 may be any tube through which a gas, liquid, gel, or other fluid may flow. For example, exudate from tissue site 105 may flow through delivery tube 135. In Figure 1, source tube 130 couples reduced pressure source 110 to a canister 140 and delivery tube 135 couples the canister 140 to the dressing 115. However, in another embodiment, reduced pressure source 110 may be directly coupled to dressing 115 using delivery tube 135.

Source tube 130 and delivery tube 135 may be made from any material. Source tube 130 and delivery tube 135 may be either flexible or inflexible. Also, source tube 130 and delivery tube 135 may include one or more paths or lumens through which fluid may flow. For example, delivery tube 135 may include two lumens. In this example, one lumen may be used for the passage of exudate from tissue site 105 to canister 140. The other lumen may be used to deliver fluids, such as air, antibacterial agents, antiviral agents, cell-growth promotion agents, irrigation fluids, or other chemically active agents, to tissue site 105. The fluid source from which these fluids originate is not shown in Figure 1. Additional details regarding the inclusion of multi-lumen tubes in reduced pressure treatment system 100 are provided below.

In one embodiment, delivery tube 135 is coupled to manifold 120 via connection member 145. Connection member 145 permits the passage of fluid from manifold 120 to delivery tube 135, and vice versa. For example, exudates collected from tissue site 105 using manifold 120 may enter delivery tube 135 via connection member 145. In another embodiment, reduced pressure treatment system 100 does not include connection member 145. In this embodiment, delivery tube 135 may be inserted directly into sealing member 125 or manifold 120 such that an end of delivery tube 135 is adjacent to or in contact with manifold 120.

Liquid, such as exudate, from tissue site 105 may flow through delivery tube 135 into canister 140. Canister 140 may be any device or cavity capable of containing a fluid, such as gases and liquids, as well as fluids that contain solids. For example, canister 140 may contain exudates from tissue site 105. Source tube 130 and delivery tube 135 may be directly connected to canister 140, or may be coupled to canister 140 via a connector, such as connector 150, as indicated by arrow 151. The canister 140 may be a flexible or rigid canister, a bag, or pouch fluidly connected to manifold 120 by delivery tube 135. Canister 140 may be a separate canister or may be operably combined with reduced pressure source 110 to collect exudate and fluids.

Reduced pressure treatment system 100 may further comprise a first pressure sensor or wound pressure sensor 155 electrically coupled to the controller 170. Wound pressure sensor 155 detects an actual reduced pressure at or proximate the tissue site 105, i.e., the tissue site pressure or wound pressure (WP). The reference to the word "wound" as part of the term wound pressure (WP) is exemplary only and does not limit the term or description herein as applying to the measurement of pressure at other types of tissue sites such as, for example, incisions or subcutaneous cavities. In one non-limiting example, wound pressure sensor 155 is a silicon piezo-resistive gauge pressure sensor. Wound pressure sensor 155 may be configured to detect the wound pressure (WP) via a control tube 160 fluidly coupled directly to the connection member 145 or indirectly by sections of the control tube 160 through the canister 140. Control tube 160 may include one or more paths or lumens through which fluid may flow.

Reduced pressure treatment system 100 may further comprise a second pressure sensor or pump pressure sensor 156 electrically coupled to the controller 170. Pump pressure sensor 156 may be configured to detect a reduced pressure at or downstream from the canister 140 by control tubes 157 and 158, respectively, i.e., the pump pressure (PP). In other words, the pump pressure sensor 156 may be fluidly coupled directly to the canister 144 by the control tube 157 or the source tube 130 by the control tube 158 to detect the pump pressure (PP). In one example embodiment, the pump pressure sensor 156 may be a silicon piezo-resistive gauge pressure sensor. In yet another example embodiment, the pump pressure (PP) may be determined by the controller 170 analyzing the DC voltage of the pump motor 114 as shown with reference to Figure 2.

Pressure sensors 155 and 156 may be located at alternate locations on or within the reduced pressure treatment system 100. Referring back to Figure 1, wound pressure sensor 155 is shown to be remote from tissue site 105. In this example, the reduced pressure at tissue site 105 may be detected directly from remotely located wound pressure sensor 155 through the control tube 160, or indirectly from the canister 140 through the section of the control tube 160 coupled to the wound pressure sensor 155. Also in this example, pump pressure sensor 156 may be directly or indirectly coupled to other remotely located components of reduced pressure treatment system 100, such as reduced pressure source 110, the canister 140, or any other illustrated component of reduced pressure treatment system 100. In another example, wound pressure sensor 155 may not require the use of control tube 160 to detect the pressure at tissue site 105. In one non-limiting example, wound pressure sensor 155 is directly coupled to manifold 120 or placed between sealing member 125 and manifold 120.

Reduced pressure treatment system 100 may also include a regulator 165 electrically coupled to the controller 170. The regulator 165 may be a valve having an input and an output wherein the input may be fluidly coupled to the external environment as indicated by arrow 166 and an output fluidly coupled to the therapeutic environment of the reduced pressure therapy system 100. For example, the output of the regulator 165 may be fluidly coupled to the canister 140 as indicated by arrow 167. In another embodiment, the output of the regulator 165 may be fluidly coupled to the control tube 160 (not shown). The regulator 165 may be any valve capable of relieving the reduced pressure in the therapeutic environment of the reduced pressure therapy system 100 thereby increasing the flow rate of fluids therein. Non-limiting examples of the regulator 165 include a pneumatic solenoid valve, a proportional valve, or a mechanical valve. In one example, the regulator 165 may be manually controlled by a caregiver. In another example, regulator 165 may be controlled by the controller 170.

In operation, the manifold 120 may be placed within, over, on, or otherwise proximate to a tissue site. The sealing member 125 may be placed over the manifold 120 and sealed to tissue near the tissue site 105. For example, the sealing member 125 may be sealed to undamaged epidermis peripheral to a wound site, i.e., the periwound. Thus, the dressing 115 can provide a sealed therapeutic environment proximate to a tissue site, substantially isolated from the external environment, and the reduced pressure source 110 can reduce the pressure in the sealed therapeutic environment. Reduced pressure applied across the tissue site through the manifold 120 in the sealed therapeutic environment can induce macrostrain and microstrain in the tissue site, as well as remove exudates and other fluids from the tissue site, which can be collected in the canister 140 and disposed of properly.

The fluid mechanics of using a reduced-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a reduced-pressure source, and conversely, the term "upstream" implies something relatively further away from a negative-pressure source. Similarly, it may be convenient to describe certain features in terms of fluid "inlet" or "outlet" in such a frame of reference. This orientation is generally presumed for purposes of describing various features and components of reduced-pressure therapy systems herein. However, the fluid path may also be reversed in some applications (such as by substituting a positive-pressure source for a reduced-pressure source) and this descriptive convention should not be construed as a limiting convention.

The term "tissue site" in this context broadly refers to a wound or defect located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness bums, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be used in certain tissue areas to grow additional tissue that may be harvested and transplanted to another tissue location.

"Negative or reduced pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure.

A negative-pressure source, such as the reduced pressure source 110, may be a reservoir of air at a reduced pressure, or may be a manual or electrically-powered device that can reduce the pressure in a sealed volume, such as a vacuum pump, a suction pump, a wall suction port available at many healthcare facilities, or a micro-pump, for example. A negative-pressure source may be housed within or used in conjunction with other components, such as processing units, alarm indicators, memory, databases, software, display devices, or user interfaces that further facilitate negative-pressure therapy. For example, the reduced pressure source 110 and the controller 106 may be housed within a therapy control unit. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

As indicated above, the applied voltage (V_{A}) provided to the DC motor 114 may be used to control the pump pressure (PP) and ultimately achieve the desired or targeted pressure at the tissue site 105. Correspondingly, the applied voltage (V_{A}) provides an indication of the pump pressure (PP) and may be monitored by the controller 170 which in turn may determine the time rate of change of the applied voltage (V_{A}) that necessarily corresponds to the time rate of change of the pump pressure (PP) in lieu of using the pump pressure sensor 156. The controller 170 may also use this computation for determining the flow rate of air between the reduced pressure source 110 and tissue site 105, i.e., the flow rate (FR). In another embodiment, the reduced pressure treatment system 100 may further comprise a sensing device (not shown) that directly measures the flow rate (FR) such as, for example, a flow-meter or a differential processor for computing the time rate of change in the difference between the wound pressure (WP) and the pump pressure (PP). Otherwise the flow rate (FR) may be determined by computing a pressure differential based on the measurements taken from two pressure sensors such as, for example, pressure sensors 155 and 156 described above.

The flow rate (FR) may be measured, for example, as cubic centimeters of air per minute (cc/min), between the reduced pressure source 110 and the tissue site 105. The flow rate (FR) provides some indication of the extent to which the dressing 115 or other components of the negative pressure system 100 might be leaking (i.e., a system leakage) that reduces the pressure at the tissue site 105 below the desired pressure targeted for therapy. For example, a high flow rate (FR) might indicate that the dressing 115 or other components of the system 100 are considered to have a "high system leakage" or a "high leakage condition" that needs to be remedied by increasing the pump pressure (PP) or adjusting the sealing around the tissue site, for example. On the other hand, a lower flow rate (FR) might indicate that the dressing 115 or other components of the system 100 are considered to have a "low system leakage" or a "low leakage condition" that requires a lower pump pressure (PP) requiring less battery power for driving the DC motor 114 at a lower rate to maintain the desired wound pressure (WP).

The controller 170 may be an integrated or separate component of the reduced-pressure treatment system 100. Controller 170 may be any device capable of processing data, such as data from wound pressure sensor 155 and/or the pump pressure sensor 156. Controller 170 may also control the operation of one or more components of reduced pressure treatment system 100, such as reduced pressure source 110, motor 114, regulator 165, pressure sensors 155 and 156, and indicator 172. The controller 170 may control and receive data from other components (not shown) of the reduced pressure source 110 including the pump 112 and the motor 114. In one embodiment, controller 170 receives and processes data, such as the wound pressure (WP) from the wound pressure sensor 155, the pump pressure (PP) from the pump pressure sensor 156, and the flow rate (FR) from monitoring the applied voltage (V_{A}) to the motor 114 as described above. The controller 170 may also control the operation of one or more components of reduced pressure treatment system 100 to manage the wound pressure (WP) at tissue site 105. In one embodiment, controller 170 may including an input for receiving a desired target wound pressure (TWP) set by a clinician or other user and may be program for processing data relating to the setting and inputting of the target wound pressure (TWP) to be applied to the tissue site 105.

In one example embodiment, the target wound pressure (TWP) may be a fixed pressure value determined by a user/caregiver as the reduced pressure target desired for therapy at the tissue site 105 and then provided as input to the controller 170. The user may be a nurse or a doctor or other approved clinician who prescribes the desired reduced pressure to which the tissue site 105 should be applied. The desired tissue site pressure will vary from tissue site to tissue site, but will generally be chosen based on the type of tissue making up the tissue site, the type of injury or wound (if any), the medical condition of the patient, and the preference of the attending physician. After selecting the desired target wound pressure (TWP), the reduced pressure source 110 is controlled to achieve the target wound pressure (TWP) for application to the tissue site 105.

Referring more specifically to Figure 3, the target wound pressure (TWP) may be set by the user in a continuous mode as indicated by solid line 401 and dotted line 402 wherein the reduced pressure is applied to the tissue site 105 until the user deactivates the reduced pressure source 110. The target wound pressure (TWP) may also be set by the user in an intermittent mode as indicated by solid lines 401, 403 and 405 wherein the wound pressure (WP) is cycled between the target wound pressure (TWP) and atmospheric pressure. For example, the target wound pressure (TWP) may be set by the user at 125 mmHg for a specified period of time (e.g., 5 min) followed by the therapy being turned off for a specified period of time (e.g., 2 min) as indicated by lines 403 by venting the tissue site 105 to the atmosphere, and then repeating the cycle by turning the therapy back on as indicated by line 405 which consequently forms a square wave pattern between the target wound pressure (TWP) level and no pressure.

It should be understood that the increase of the wound pressure (WP) at the tissue site 105 from ambient pressure to the target wound pressure (TWP) is not instantaneous, but rather limited depending on the type of therapy equipment and the dressing. For example, the reduced pressure source 110 and the dressing 115 may have an initial rise time as indicated by the dashed line 407 that may vary depending on the type of dressing and therapy equipment being used. For example, the initial rise time for one therapy system may be in the range between about 20-30 mmHg/second or, more specifically, equal to about 25 mmHg/second, and in the range between about 5-10 mmHg/second for another therapy system. When operating in the intermittent mode, the repeating rise time, line 405, may be substantially equal to the initial rise time, line 407.

The target pressure may also be a variable target pressure (VTP) controlled or determined by controller 170 that varies in a dynamic pressure mode. For example, the variable target pressure (VTP) may vary between a maximum and minimum pressure value that may be set as an input by a user as the range of reduced pressures desired for therapy at the tissue site 105. The variable target pressure (VTP) may also be processed and controlled by controller 170 that varies the target wound pressure (TWP) according to a predetermined waveform such as, for example, a sine waveform or a saw-tooth waveform or a triangular waveform, that may be set as an input by a user as the predetermined or time-varying reduced pressures desired for therapy at the tissue site 105. For example, the variable target pressure (VTP) may be a reduced pressure that provides an effective treatment by applying reduced pressure to tissue site 105 in the form of a triangular waveform varying between 50-125 mmHg with a rise time set at +25 mmHg/min and a descent time set at -25 mmHg/min. In another embodiment of a reduced-pressure therapy system 100, the variable target pressure (VTP) may be a reduced pressure that applies reduced pressure to tissue site 105 in the form of a triangular waveform varying between 25-125 mmHg with a rise time set at a rate of +30 mmHg/min and a descent time set at -30 mmHg/min. Again, the type of system and tissue site determines the type of reduced pressure therapy to be used.

After selecting the target wound pressure (TWP), the reduced pressure source 104 is operated to achieve the desired pressure at the wound site 105 by controlling pressure (PP). In many cases, the reduced pressure source 110 to be operated at a higher pump pressure (PP) than that of the target wound pressure (TWP) due to pressure losses between the reduced pressure source 110 and the tissue site 105. Moreover, the head pressure of exudates and other fluids within the conduits may result in a reduction of vacuum pressure at the tissue site 105. The height of the canister 140 above the tissue site 105 may determine the amount of head pressure imposed on the tissue site 105 by fluid in the conduits. For exudates and fluids with a density similar to water, the head pressure imposed by one foot of fluid is almost 25 mmHg. Some fluids withdrawn from the tissue site 105 may be even heavier or more viscous than water, and therefore have a more pronounced effect on pressure losses at the tissue site 105.

Referring to Figure 4 as an example of the potential losses caused by the weight of fluid in the conduits, the target wound pressure (TWP) prescribed for a particular tissue site may be -125 mm Hg wherein the wound pressure (WP) varies as the reduced pressure is applied to the tissue site 105. (It should be understood that the steady sinusoidal variations of the wound pressure (WP) shown in Figure 4 are only explanatory and not representative of the actual variations of the wound pressure (WP) under normal operational conditions such as, for example, the variations shown in Figures 5A and 5B.) If the canister 140 is positioned two feet above the tissue site 105, and if the delivery tube 135 between the canister 140 and tissue site 105 is completely full of fluid, the head pressure imposed by that fluid could create a pressure differential (δP) of approximately 50 mmHg. This particular example occurs when a tissue site is located on a lower extremity of a patient such as a foot and the canister 140 is mounted near or above the patient's head (e.g., on an IV pole when the patient is in a wheelchair). Therefore, if the head pressure of fluid in the delivery tube 135 is approximately 50 mmHg, the pump 112 needs to provide a pump pressure (PP) rising to a maximum pump pressure value (PPmax) of approximately 185 mmHg and dropping to a minimum pump pressure value (PPmin) of approximately 165 mmHg (a median target pump pressure (TPP) of approximately 175 mmHg) to yield a target wound pressure (TWP) at the tissue site 105 of approximately 125 mmHg.

The controller 170 may also be programmed and controlled by a user to maintain the target wound pressure (TWP) within an acceptable range of pressures. For example, if the target wound pressure (TWP) is set at 125 mmHg as the desired therapeutic pressure for the tissue site 105, a user may desire that the wound pressure (WP) varies by no more than ±10 mmHg from the desired target wound pressure (TWP) so that the wound pressure (WP) is controlled between a minimum wound pressure value (WPmin) of 115 mmHg and a maximum wound pressure value (WPmax) of 135 mmHg, i.e., a differential wound pressure range (δWP) of about 20 mmHg. Therefore, assuming for this example that there is a head pressure approximately 50 mmHg as described above, the pump pressure (PP) must also be variable by ±10 mmHg from the target pump pressure (TPP) so that the pump pressure (PP) may be varied in a range extending from the minimum pump pressure value (PPmin) of approximately 165 mmHg to the maximum pump pressure value (PPmax) of approximately 185 mmHg, i.e., a differential pump pressure (δTTP) of about 20 mmHg. Controlling the pump pressure (PP) to stay within this range indirectly maintains the wound pressure (WP) within a range extending from the minimum wound pressure value (WPmin) of approximately 115 mmHg to the maximum wound pressure value (WPmax) of approximately 135 mm.

Referring to Figure 5A as an example of wound pressure (WP) variations under normal operating conditions in contrast to the example illustrated in Figure 4, the pressure differential (δP) between the pump pressure (PP) and the wound pressure (WP) is the result of a fairly high flow rate (FR) of approximately 300 cc/min (a high leakage condition) in the dressing 115 and other components in the system. In this example, the wound pressure (WP) is being controlled to cycle between approximately 135 mmHg and 115 mmHg as described above by providing a pump pressure (PP) that rises to a maximum pump pressure value (PPmax) of approximately 155 mmHg and drops to a minimum pump pressure value (PPmin) of approximately 120 mmHg to yield a target wound pressure (TWP) at the tissue site 105 of approximately 125 mmHg. Thus, the pressure differential (δP) is approximately 15 mmHg which is much less than the pressure differential (δP) of approximately 50 mmHg resulting from the head pressure in the example associated with Figure 4 above. Figure 5B illustrates yet another example wherein the pressure differential (δP) between the pump pressure (PP) and the wound pressure (WP) is the result of a lower flow rate (FR) of approximately 50 cc/min (a low leakage condition) in the dressing 115 and other components in the system. In this example, the wound pressure (WP) is again being controlled to cycle between approximately 135 mmHg and 115 mmHg by providing a pump pressure (PP) that rises to a maximum pump pressure value (PPmax) of 140 mmHg and drops to a minimum pump pressure value (PPmin) of 115 mmHg to yield a target wound pressure (TWP) at the tissue site 105 of approximately 125 mmHg. Thus, the pressure differential (δP) is approximately 5 mmHg which is even less than the pressure differential in the previous example.

The controller 170 may also comprise a bang-bang controller (not shown) which is also referred to as an on-off controller, or a hysteresis controller. The bang-bang controller is a feedback controller that switches abruptly between two states, e.g., between on and off. Essentially, the bang-bang controller may apply an all-or-nothing form of control. A bang-bang controller may be used to generate the pressure variations described generally above in conjunction with Figures 5A and 5B. Continuing with that general description, the bang-bang controller may operate in one mode as follows. For example, when the wound pressure (WP) drops too low to the minimum wound pressure value (WPmin), the reduced pressure pump 112 is turned on with an applied voltage (V_{A}) greater than the stall voltage, i.e., the bang-bang on voltage (V_{ON}) at a start time (tₒₙ), to increase the pump pressure (PP) to the maximum pump pressure (PPmax). Although an increase in the pump pressure (PP) may slightly lag the application of the applied voltage (V_{A}), the increasing pump pressure (PP) eventually causes the wound pressure (WP) to increase as well as shown at time t₁. The bang-bang on voltage (V_{ON}) continues to be applied until the pump pressure (PP) reaches the maximum pump pressure value (PPmax) or the wound pressure (WP) reaches the maximum wound pressure value (WPmax), whichever occurs first. When either one of these maximum values is reached or exceeded, the reduced pressure pump 112 is turned off at an off time (t_{off}) so that no pump pressure (PP) is applied allowing the residual pressure in the reduced pressure therapy system 100 to decrease as a result of the leakage in the system. The reduced pressure pump 112 remains off until the wound pressure (WP) is again less than or equal to the minimum wound pressure value (WPmin) or the pump pressure (PP) is less than or equal to the minimum pump pressure value (PPmin), whichever occurs first.

The bang-bang controller switches between these two states wherein the reduced pressure pump 112 is turned on when the wound pressure or the pump pressure drops too low in a descending mode and turns the reduced pressure pump 112 off when the wound pressure or pump pressure rises too high in an ascending mode. Referring more specifically to Figure 5B, the bang-bang controller allows the wound pressure (WP) to oscillate proximate the target wound pressure (TWP) of 125 mmHg as contained between the two limits that a user programs into the controller 170, e.g., the minimum wound pressure value (WPmin) of 115 mmHg and the maximum wound pressure value (WPmax) of 135 mmHg. The wound pressure (WP) is not pulled back within the wound pressure range (δWP) of 20 mmHg unless the wound pressure (WP) exceeds either one of these limits. The bang-bang controller keeps the wound pressure (WP) substantially within this range because the bang-bang controller does not need to overcompensate for leakage in a low-leakage environment.

The controller 170 may also include a PID controller (not shown) that provides a control loop feedback mechanism that calculates an error value as the difference between a measured process variable and a desired set-point or target, in this case the wound pressure (WP) and the corresponding target wound pressure (TWP) at the wound site 105, respectively. PID controllers are well-known by those skilled in the art as providing proportionality information, historical information, and time rate of change information to maintain the wound pressure (WP) close to the target pressure (TP). The PID summation is used to adjust the process, in this case the reduced pressure therapy process, by a control element such as the power or voltage supplied to a DC motor, i.e. the applied voltage (V_{A}), which is directly related to the pump pressure (PP) as described above. The applied voltage (V_{A}) may be varied as described above by adjusting the pulse-width modulation to achieve the desired pump pressure (PP) necessary to compensate for the leakage of the dressing 115 and/or the pressure head referred to above. The response of the PID controller is dependent on the responsiveness of the controller to an error, the degree to which the controller overshoots the set-point, e.g., the target pressure (TP), and the degree of system oscillation, e.g., the degree of oscillation of the wound pressure (WP) within the acceptable range described above. Although a preferred embodiment of the PID controller is a digital controller, the PID controller may also be an analog controller or a simple RC circuit. The analog or digital PID controller may be implemented in software as part of a program logic controller.

After the wound pressure sensor 155 measures the wound pressure (WP), the PID controller adjusts the pump pressure (PP) by supplying the applied voltage (V_{A}) necessary for adjusting the wound pressure (WP) back to the target pressure (TP), i.e., the pump pressure correction (δPP). The pump pressure correction (δPP) is the additional pressure needed to maintain the wound pressure (WP) at the desired target pressure (TP), e.g., 125 mmHg, and may be calculated every few seconds. Consequently, the PID control varies the applied voltage (V_{A}) to the DC motor 114 to achieve a pump pressure (PP) between a minimum pump pressure value (PPmin) and a maximum pump pressure value (PPmax) which maintains the wound pressure (WP) proximate the target wound pressure (TP).

Referring more specifically to Figure 6 as an example of maintaining the wound pressure (WP) under normal operating conditions of a PID controller in contrast to the example illustrated in Figure 4, the pressure differential (δP) between the pump pressure (PP) and the wound pressure (WP) is the result of different leakage rates (LR) as illustrated by the three examples including the first pump pressure (PP1), the second pump pressure (PP2), and the third pump pressure (PP3). In the first example, the first pump pressure (PP1) has a relatively large pressure differential (δP1) of approximately 15-16 mmHg resulting from a fairly high flow rate (FR) of approximately 350 cc/min. The first pressure (PP1) is varied by the PID controller between a maximum pump pressure value (PPmax) and a minimum pump pressure value (PPmin) to maintain the wound pressure (WP) at the target wound pressure (TWP) of 125 mmHg. In other words, the PID controller varies the first pump pressure (PP1) between 140 mmHg and 141 mmHg to maintain the wound pressure (WP) at the target wound pressure (TWP) of 125 mmHg. In the second example, the second pump pressure (PP2) also has a relatively large pressure differential (δP2) of approximately 11-12 mmHg resulting from a fairly high flow rate (FR) of approximately 250 cc/min and is varied by the PID controller between a maximum pump pressure value (PPmax) and a minimum pump pressure value (PPmin) to maintain the wound pressure (WP) at the target wound pressure (TWP) of 125 mmHg. In other words, the PID controller varies the second pump pressure (PP2) between 136 mmHg and 137 mmHg to maintain the wound pressure (WP) at the target wound pressure (TWP) of 125 mmHg. Fundamentally, the difference between these two examples is that the higher flow rate (FR) requires a larger pressure differential (δP) to maintain the wound pressure (WP) at the same target pressure (TP). The third example illustrates the same difference wherein the third pump pressure (PP3) also has a much smaller pressure differential (δP3) of approximately 4-5 mmHg resulting from a lower flow rate (FR) of approximately 100 cc/min and is varied by the PID controller between 129 mmHg and 130 mmHg to maintain the wound pressure (WP) at the target wound pressure (TWP) of 125 mmHg.

Unlike the bang-bang controller, the PID controller does not switch the reduced pressure pump 112 on and off, but rather continuously controls the application of the pump pressure (PP) between the maximum and minimum pressure values, (PPmax) and (PPmin), to maintain the wound pressure (WP) at a relatively constant level, e.g., at a target wound pressure (TWP) all of 125 mmHg as shown by the dashed line, rather than allowing it to vary between a maximum and minimum pressure value, (WPmax) and (WPmin) as shown with the bang-bang controller. Therefore, the extent to which the pump pressure (PP) drops towards the minimum pump pressure value (PPmin), the more that the PID controller increases the applied voltage (V_{A}) being provided to the DC motor 114. Correspondingly, the further the wound pressure (WP) varies from the target pressure (TP), the more the PID controller responds by adjusting the applied voltage (V_{A}) being provided to the DC motor 114. The action taken to increase or decrease the applied voltage (V_{A}) is proportional to the degree that the wound pressure (WP) provided by the reduced pressure system diverges from the target wound pressure (TP). The PID controller continuously operates in order to keep the wound pressure (WP) as close to the target wound pressure (TP) as possible, especially for high leakage rates (LR). Consequently, the PID controller causes the reduced pressure therapy system 100 to run smoother than the bang-bang controller as shown when comparing the wound pressure (WP) variations of Figures 6 and 5, respectively, because the PID controller maintains the wound pressure (WP) closer to the target wound pressure (TP) on average, while the bang-bang controller allows the wound pressure (WP) to oscillate between the two limits as described above.

When the flow rate (FR) is small enough to indicate a low leakage condition, e.g., when the pump pressure (PP) or the wound pressure (WP) is decreasing at a very slow rate toward their respective minimum pressure values, i.e., (PPmin) or (WPmin), the bang-bang controller may provide a sufficiently smooth wound pressure (WP) during treatment while conserving battery power and reducing noise by virtue of the reduced pressure pump 112 being intermittently turned off during the same treatment period. For example, the DC motor 114 and pump 112 are turned off for a significant percentage of time during the one minute period shown in Figure 5B, but run continuously when the PID controller is operative as shown in Figure 6. Hence, it is desirable to keep the bang-bang controller running during treatment sessions as much as possible for low leakage conditions such as, for example, when the flow rate (FR) is less than or equal to a fixed target flow rate (TFR) which represents a low leak condition, but switch to the PID controller when the flow rate (FR) is greater than the fixed target flow rate (TFR) which represents a high leak condition. Consequently, another example embodiment of the controller 170 includes both the PID controller and the bang-bang controller, i.e., a hybrid controller, and additional processing that switches between them depending on the degree of leakage of the reduced pressure therapy system 100 regardless of the location of the leaks or leakage.

Thus, the controller 170 may be programmed to use the bang-bang controller in conjunction with the PID controller operating as described above to enable or disable the PID controller depending on a specific switching condition relating to the amount of system leakage created by the dressing 115 or other components of the reduced pressure therapy system 100 that affect the flow rate (FR). Using such a hybrid controller would be preferable to utilizing only a PID controller which runs continuously during the continuous control mode as described above (or the enabled portions of an intermittent control mode as described above) to more tightly maintain the wound pressure (WP) at the target wound pressure (TP), but may continually generate noise and more rapidly drain the battery driving the motor 114. The hybrid controller may engage the bang-bang controller so that the DC motor 114 is turned on and off to conserve battery power and reduce noise generated by the pump 112 during therapy treatments. The controller 170 may further include an input for a user/caregiver to set one or more target flow rates (TFR).

The user/caregiver may set the target flow rates (TFR) as the switching condition for determining whether the dressing 115 or other components are in a high leakage state or a low leakage state. If the flow rate (FR) is greater than the fixed target flow rate (TFR), i.e., a high leak condition, the bang-bang controller is disabled so that the PID controller takes over in order to keep the wound pressure (WP) as close to the target wound pressure (TP) as possible. However, if the flow rate (FR) is less than or equal to the fixed target flow rate (TFR), i.e., a low leak condition, the bang-bang controller is enabled to contain the wound pressure (WP) within the differential wound pressure (δWP) range while conserving battery power and reducing noise from the pump 112. For example, the fixed target flow rate (TFR) may be 65 cc/min. As indicated above, it is desirable to keep the bang-bang controller running as much as possible during treatments when the dressing 115 is in a low leakage condition. For example, the controller 170 may engage the bang-bang controller when the flow rate (FR) is less than or equal to the fixed target flow rate (TFR), but switch back to the PID controller when the flow rate (FR) is greater than the fixed target flow rate (TFR) as a result of additional leakage that develops in the dressing 115 because the patient moving around which ultimately creates a high leak condition.

In another embodiment, the bang-bang controller may have a dual target flow rate (TFR) capability wherein the controller 170 further includes an input for a user to set two target flow rates (TFR) as switching conditions for determining whether the dressing 115 or other components are in a high leakage state or a low leakage state: an ascending target flow rate (TFR_{A}) when the bang-bang controller is enabled with an increasing flow rate (FR) and a descending target flow rate (TFR_{D}) when the PID controller is enabled with a decreasing flow rate (FR). In one embodiment, both the ascending target flow rate (TFR_{A}) and the descending target flow rate (TFR_{D}) are greater than the fixed target flow rate (TFR) so that the controller 170 switches more quickly from the PID controller to the bang-bang controller and more slowly from the bang-bang controller to the PID controller. For example, the ascending target flow rate (TFR_{A}) and the descending target flow rate (TFR_{D}) may both be set to about 80 cc/min which is higher than the fixed target flow rate (TFR) of 65 cc/min in the previous example. In yet another embodiment, the ascending target flow rate (TFR_{A}) may also be greater than the descending target flow rate (TFR_{D}) so that the controller 170 switches even more quickly from the PID controller to the bang-bang controller and even more slowly from the bang-bang controller to the PID controller. In this case, the controller 170 favors the benefits derived from using the bang-bang controller as opposed to the deficiencies associated with the continuous operation of the PID controller. For example, the ascending target flow rate (TFR_{A}) may be 75 cc/min and the descending target flow rate (TFR_{D}) may be about 85 cc/min. If the PID controller is currently enabled in a high leak condition wherein the flow rate (FR) is decreasing, the descending target flow rate (TFR_{D}) would be set at 85 cc/min rather than 65 cc/min so that the controller 170 switches more quickly from the PID controller to enable the bang-bang controller. Alternatively, if the bang-bang controller is enabled in a low leak condition wherein the flow rate (FR) is increasing, the ascending target flow rate (TFR_{A}) would be set at 75 cc/min rather than 65 cc/min so that the controller 170 switches more slowly to disable the bang-bang controller.

In one embodiment, controller 170 may provide an output signal to the indicator 172 to emit a visual and/or audible signal in response to the wound pressure (WP) at tissue site 105, as measured by wound pressure sensor 155, being nonresponsive to increasing the pump pressure (PP). For example, the indicator may be a light emitting diode (LED) that provides a visual signal. In this embodiment, indicator 172 illuminates in response to the wound pressure (WP) at tissue site 105 being nonresponsive to an increasing pump pressure. In another embodiment, indicator 180 is a sound emitting device, such as a speaker. In this embodiment, indicator 172 emits a sound in response to the wound pressure (WP) at tissue site 105 being nonresponsive to an increasing pump pressure. The controller 170 may provide other output signals indicating whether the negative pressure therapy system is in a low or high leak condition.

Although most reduced pressure therapy systems have some system leakage, improvements in the dressings and other components of the system have greatly reduced system leakages even lower than the low system leakages such as the target flow rates (TFRs) referred to above. For example, sealing members such as sealing member 125 and the adhesives for attaching sealing members to the peritissue have been improved so that they are more airtight and significantly reduce the system leakage from the external environment into the therapeutic environment. In some cases, the wound pressure (WP) might already be equal to the targeted wound pressure (TWP), while the flow rate (FR) at the tissue site 105 may range from less than 50 cc/min down to zero. Flow rates (FR) less than the target flow rates (TFRs) are often too low for healing the wound. Some minimal level of fluid flow is needed at the tissue site to promote adequate healing. For example, a minimum level of fluid flow is needed to remove exudates from the tissue site. If the flow rate (FR) falls below a minimum flow rate (MinFR) necessary for the proper healing of a wound [that in some embodiments is less than the target flow rate (TFR)], it would be desirable to measure the flow rate (FR) associated with the system leakage and increase the flow rate (FR) over and above the system flow rate to an acceptable level (i.e., an "induced leakage") for healing the wound at the tissue site 105 in an "induced leakage mode." In some embodiments, the minimum flow rate (MinFR) may be dependent upon or equal to the target flow rate (TFR). In yet other embodiments, the minimum flow rate (MinFR) more dependent on the specific type of dressing and/or the type of wound that needs to be healed.

In some embodiments, the controller 170 may be configured to determine the flow rate (FR) associated with the system leakage and compare it to the minimum flow rate (MinFR) that is desired for a specific type of dressing and/or wound type. The controller 170 may be further configured to increase the leakage by opening the regulator 165 if the flow rate (FR) is less than the minimum flow rate (MinFR). When the regulator 165 opens to induce leakage from the external environment into the therapeutic environment to increase the flow rate (FR) of fluids within the therapy system and ultimately at the tissue site 105, the controller 170 may be further configured to regulate the flow rate (FR) as it increases toward the minimum flow rate (MinFR) and then maintains the flow rate (FR) above the minimum flow rate (MinFR). In some embodiments, the controller 170 may be configured further to include an algorithm for inducing leakage into the therapeutic environment in a controlled fashion. For example, the algorithm may include a ramp function, linear or nonlinear, to increase the flow rate (FR) to the minimum flow rate (MinFR). The algorithm may further comprise a sinusoidal function that maintains the flow rate (FR) above the minimum flow rate (MinFR) by varying the amplitude and/or the frequency. The algorithm may further comprise a digital function that varies the flow rate (FR) by varying the amplitude, the frequency, and/or the duty cycle.

As indicated above, the system 100 may have a system leakage stemming from the dressing and/or other components of the system and the wound itself. Thus, different reduced pressure therapy systems may have different system leakage values depending on these factors. For example, one system may have a low system leakage of 50 cc/min while another system may have a low leakage condition at 100 cc/min. In some embodiments, the low leakage condition may be set as the minimum flow rate (MinFR) which also may vary from 50-100 cc/min, for example. The controller 170 may be configured to open the regulator 165 for inducing leakage into the therapy system 100 when the flow rate (FR) of fluids within the therapy system drops below the minimum flow rate (MinFR).

In one example, a dressing as a minimum flow rate (MinFR) requirement of 100 cc/min, but the controller measures the flow rate (FR) of 50 cc/min wherein the fluids need to be evacuated to a canister two feet above the dressing. The effects of gravity and a thicker fluid because of the exudates, could cause the fluid flow of such a small leak to remain static in the dressing. However, the controller detects that the flow rate (FR) is lower than the minimum flow rate (MinFR) requirement of 100 cc/min, and opens and controls a regulator to induce leakage of airflow into the therapeutic environment of the dressing. The algorithm may further comprise a digital function that varies the flow rate (FR) by varying the amplitude, the frequency, and/or the duty cycle. For example, a minimum flow rate (MinFR) requirement of 100 cc/min equals a drop of 1.0mmHg/10 seconds and a flow rate (FR) of 50 cc/min equals a drop of 0.5mmHg/10 seconds. If the algorithm includes a digital function for example, the frequency may be increased to one cycle/5 seconds or 12 cycles/minute with a duty cycle of 20%/cycle, i.e., 1 second width/cycle. This would cause the wound pressure (WP) at the tissue site to decrease (alternatively, cause the pressure decay to increase) and the flow rate (FR) to increase to at least 100 cc/min, thereby overcoming the force of gravity and allowing the canister to be moved 2 feet above the dressing. The bang-bang controller in conjunction with the PID controller may then operate as described above to adjust the wound pressure (WP) as required.

Referring now to Figure 7, an example embodiment of a method or process for controlling the wound pressure (WP) as implemented on a controller such as, for example, the controller 170 as described above or, alternatively, on another example embodiment of the controller 170. The controller 170 and other components may implement this process as described above according to a therapy loop 700 illustrated as the flowchart in Figure 7. The therapy loop 700 includes a therapy algorithm 703 for selecting the appropriate controller, i.e., the PID controller or the bang-bang controller, for controlling the delivery of reduced pressure to the tissue site while conserving power and reducing noise from the pump 112 and the motor 114 at the same time. The controller 170 first checks to see if the negative pressure therapy system 100 has been turned on at 705 so that if the negative pressure therapy system 100 is not on, the applied voltage (V_{A}) is set to 0 V at 707 and applied to the motor 114 as a new motor voltage (V_{M}) at 709 so that the motor 114 is not running. If the negative pressure therapy system 100 is turned on, the controller 170 checks to determine whether enough time has elapsed at 711 to engage the therapy algorithm 703, i.e., the duty cycle therapy time (t_{DC}). The duty cycle of the therapy algorithm 703 may be, for example, about 50 ms. Thus, if less than 50 ms transpires since the therapy algorithm 703 was last calculated, the motor voltage (V_{M}) remains set at the previously applied voltage (V_{A}) at 709. The duty cycle of the therapy loop 700 itself may be, for example, 10 ms without engaging the therapy algorithm 703. However, if more than 50 ms have transpired, the controller 170 recalculates the therapy algorithm 703 and proceeds to check the current wound pressure (WP) and/or the pump pressure (PP) at 713 with respect to their corresponding maximum and minimum wound pressure and pump pressure values as described above, i.e., (WPmax) and (WPmin), and (PPmax) and (PPmin), respectively.

The therapy algorithm 703 begins by determining whether the bang-bang controller is active or not at 713. If the PID controller is engaged and the bang-bang controller is not, a local pump pressure (PPL) is set at a current pump pressure (PPC) at 715. As described above, the PID control adjusts the applied voltage (V_{A}) to the DC motor 114 to achieve a pump pressure (PP) between the minimum pump pressure value (PPmin) and the maximum pump pressure value (PPmax) to maintain the wound pressure (WP) proximate the target wound pressure (TP). Referring back to Figure 6 as an example, the PID controller varies the first pump pressure (PP1) between 140 mmHg and 141 mmHg to maintain the wound pressure (WP) at the target wound pressure (TWP) of 125 mmHg and continues to control the pump pressure (PP) during a high leakage condition. The controller 170 determines the value of applied voltage (V_{A}) corresponding to the current pump pressure (PPC) at 717 and applies that voltage as the motor voltage (V_{M}) at 709. However, if the bang-bang controller is engaged or active as shown in Figure 5B, the therapy algorithm 703 determines whether the bang-bang controller is ascending or descending at 719.

When the wound pressure (WP) drops too low in the descending mode, e.g., below the minimum wound pressure value (WPmin) as described above, the reduced pressure pump 112 is turned on with an applied voltage (V_{A}) greater than the stall voltage, i.e., the bang-bang on voltage (V_{ON}), to increase the pump pressure (PP) to the maximum pump pressure (PPmax) in the ascending mode. The bang-bang on voltage (V_{ON}) continues to be applied until the pump pressure (PP) reaches the maximum pump pressure value (PPmax) as shown, for example, at 501 and 503, or the wound pressure (WP) reaches the maximum wound pressure value (WPmax) as shown, for example, at 502 and 504, whichever occurs first. When the wound pressure (WP) is in the ascending mode, the therapy algorithm 703 sets the local target wound pressure (TPL) at the target wound pressure (TP) plus a hysteresis value (H) at 723. The hysteresis value (H) is the maximum amount of pressure that the wound pressure (WP) should increase above the target wound pressure (TP) when in the ascending mode before the controller 170 turns off the pump 112 to protect the tissue site 105 from an excessive amount of reduced pressure that could be damaging. The hysteresis value (H) sets the upper limit above the target wound pressure (TP) which is the maximum pressure value (WPmax). For example, if the hysteresis value (H) is 10 mmHg, the maximum wound pressure value (WPmax) is set at 135 mmHg as shown in Figure 5B. Because the wound pressure (WP) normally trails the ascending pump pressure (PP) as shown by the wound pressure peaks at 502 and 504, and the pump pressure peaks at 501 and 503, the wound pressure (WP) is normally less than the maximum wound pressure value (WPmax), e.g., about 132 mmHg at 505 and 506, when the pump pressure (PP) hits the maximum pump pressure value (PPmax), e.g., about 140 mmHg at 501 in 503. Consequently, the controller 170 allows the bang-bang controller to continue regulating the application of reduced pressure, but does turns off the pump 112 in the descending mode of the reduced pressure cycle.

Correspondingly, when the wound pressure (WP) rises too high in the ascending mode, e.g., above the maximum wound pressure value (WPmax) or the maximum pump pressure value (PPmax) as described above, the reduced pressure pump 112 is turned off so that no pump pressure (PP) is applied allowing the residual pressure in the reduced pressure therapy system 100 to decrease in the descending mode as a result of the leakage in the system. The reduced pressure pump 112 remains off until the wound pressure (WP) is again less than or equal to the minimum wound pressure value (WPmin) is shown, for example, at 508, or the pump pressure (PP) is less than or equal to the minimum pump pressure value (PPmin) as shown, for example, that 507, whichever occurs first. When the wound pressure (WP) is in the descending mode as described above, the therapy algorithm 703 sets the local target wound pressure (TPL) at the target wound pressure (TP) minus the hysteresis value (H) at 721. The hysteresis value (H) is the minimum amount of pressure that the wound pressure (WP) should decrease below the target wound pressure (TP) when in the descending mode before the controller 170 determines that the flow rate (FR) has increased to a flow rate that is large enough to require the PID controller to maintain the wound pressure (WP) closer to the target wound pressure (TP) as described above. Thus, the hysteresis value (H) also sets the lower limit below the target wound pressure (TP) which is the minimum pressure value (WPmin). For example, if the hysteresis value (H) is 10 mmHg, the minimum wound pressure value (WPmin) is set at 115 mmHg as shown in Figure 5B. Because the pump pressure (PP) normally follows the descending wound pressure (WP) as shown between the pump and wound pressure peaks at 501 and 502, respectively, and the pump and wound pressure minimums that 507 and 508, respectively, the bang-bang controller turns the pump 112 back on at 507 after which the wound pressure (WP) begins to increase again in the ascending mode. Consequently, the controller 170 allows the bang-bang controller to continue regulating the application of reduced pressure, and does so by turning on the pump 112 in the ascending mode of the reduced pressure cycle. The bang-bang controller allows the wound pressure (WP) to effectively oscillate around the target wound pressure (TWP) of 125 mmHg as contained between the two limits that may be programmed into the controller 170 separately using the minimum wound pressure value (WPmin) of 115 mmHg and the maximum wound pressure value (WPmax) of 135 mmHg, or using the hysteresis value (H). In either case, the bang-bang controller maintains the wound pressure (WP) within a wound pressure range (δWP), e.g., a wound pressure range (δWP) of 20 mmHg.

After the therapy algorithm 703 sets the motor voltage (V_{M}) to equal the applied voltage (V_{A}) at 709 to reenter the therapy loop 700, the therapy loop 700 then reads the current flow rate (FR) measured by the controller 170 at 725 and determines whether or not the current flow rate (FR) is less than the target flow rate (TFR) at 727. If the flow rate (FR) is less than the target flow rate (TFR) indicating a low leakage condition as described above, then the bang-bang controller stays on or is enabled as indicated at 729. However, if the flow rate (FR) is greater than or equal to the target flow rate (TFR) indicating a high leakage condition as described above, then the bang-bang controller stays off or is disabled as indicated at 731. Finally, the therapy loop 700 checks to see if the negative pressure wound therapy system 100 has been turned off at 733 and, if not, continues the therapy loop as indicated at 735. If the negative pressure therapy system 100 has been turned off, the therapy loop ends at 737.

Referring now to Figure 8, an example embodiment of a method or process for controlling the flow rate (FR) as implemented on a controller such as, for example, on the controller 170 as described above or, alternatively, on another example embodiment of the controller 170. The controller 170 and other components may implement this process as described above according to a flow rate (FR) loop 800 illustrated as the flowchart in Figure 8. The flow rate (FR) loop 800 is executed periodically to ensure that the flow rate (FR) has not diminished below the minimum flow rate (MinFR). The flow rate (FR) loop 800 commences at 802 with reading the flow rate (FR) and then by comparing the flow rate (FR) to the minimum flow rate (MinFR) at 804. If the flow rate (FR) is not less than the minimum flow rate (MinFR) at 804, then negative pressure therapy continues at 806 which in some embodiments may return to the therapy loop at 700. If the flow rate (FR) is less than the minimum flow rate (MinFR) at 804, then the flow rate (FR) loop 800 generates a low-leak signal (not shown) and proceeds to induce leakage at 808 as described in more detail above in response to the low-leak signal. The flow rate (FR) loop 800 reads the flow rate (FR) again (not shown) and compares the new flow rate (TF) to the minimum flow rate (MinFR) at 810. If the new flow rate (FR) is still less than the minimum flow rate (MinFR) at 810, then the flow rate (FR) loop 800 continues to induce leakage at 808 as described in more detail above. That cycle continues until the new flow rate (FR) is no longer less than, but rather greater than, the minimum flow rate (MinFR) at 810. The negative pressure therapy continues at 806 and onto the therapy loop 700 in a preferred embodiment, but the therapy loop 700 should commence only after the system has reached equilibrium within the desired wound pressure (WP) range in some embodiments.

The systems, apparatuses, and methods described herein may provide significant advantages. For example, PID control algorithms constantly adjust a negative-pressure source to maintain pressure within a specified tolerance, which can be inefficient in low-leak applications, drawing more power than a simple hysteresis control algorithm. Conversely, a hysteresis algorithm can work well in low-leak applications and uses relatively little power, but can cause a negative-pressure source to turn off and on frequently in high-leak applications, which can be noisy and increase power consumption. Hybrid control, as described herein, can combine the benefits of PID and hysteresis control algorithms to minimize power consumption and noise. If a negative-pressure therapy application has a low-leak, for example, a hybrid control algorithm can select a hysteresis control algorithm to minimize power consumption. If the application changes or develops a higher leak, a hybrid control algorithm can switch to a PID control algorithm to minimize noise.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context.

## Claims

1. A system for promoting healing of a wound at a tissue site, comprising:
a dressing (115) including a porous pad (120) configured to be positioned at the tissue site and adapted to be covered by a drape (125) to form a therapeutic environment separated from the external environment for maintaining a wound pressure (WP);
a negative-pressure source (110) including a pump (112) adapted to generate a pump pressure (PP) and further adapted to be fluidly coupled to the porous pad (120) for applying negative pressure to the tissue site;
a flow rate sensor having an input fluidly coupled between the pump (112) and the porous pad (120) and an output for providing a flow-rate signal representing a flow rate (FR) of fluids indicative of leakage between the pump (112) and the porous pad (120);
a controller (170) having an input coupled to the output of the flow rate sensor and an output, wherein the controller (170) is configured to (i) determine a flow rate (FR) based on the flow-rate signal, (ii) compare the flow rate (FR) to a minimum flow rate (MinFR), and (iii) generate a low-leak signal at the output of the controller (170) when the flow rate (FR) is less than the minimum flow rate (MinFR); and
a regulator (165) coupled to the output of the controller (170) and adapted to increase the flow rate (FR) by increasing the leakage into the therapeutic environment in response to the occurrence of a low-leak signal.

2. The system of claim 1, wherein the flow rate sensor comprises a first pressure sensor (156) having a first input for sensing the pump pressure (PP) and a first output for providing a signal indicative of the pump pressure (PP), and a second pressure sensor (155) having in a second input for sensing the wound pressure (WP) and a second output for providing a signal indicative of the wound pressure (WP), and wherein the controller (170) is electrically coupled to the first output of the first pressure sensor (156) and the second output of the second pressure sensor (155) and is further configured to determine the flow rate (FR) based on the difference between pump pressure (PP) and the wound pressure (WP).

3. The system of claim 1, wherein the regulator (165) is configured to increase the leakage by venting gases from the external environment to the therapeutic environment.

4. The system of claim 3, wherein the regulator (165) is a control valve.

5. The system of claim 3, wherein the leakage has a first leakage threshold and a second leakage threshold.

6. The system of claim 3, wherein the regulator (165) is configured further to increase the leakage from zero to a first leakage threshold.

7. The system of claim 6, wherein the regulator (165) is configured further to increase the leakage by venting gases at a fixed rate.

8. The system of claim 6, wherein the regulator (165) is configured further to increase the leakage by venting gases at a variable rate.

9. The system of claim 8, wherein the regulator (165) is configured further to vary the amplitude, frequency, or duty cycle of the variable rate.

10. The system of claim 1, wherein the minimum flow rate (MinFR) is less than about 50 cc/minute or less than about 100 cc/minute.

11. The system of claim 1, wherein the minimum flow rate (MinFR) comprises two or more target flow rates.

12. The system of claim 14, wherein the controller (170) is configured further to adjust the flow rate (FR) based on a first target flow rate and a second target flow rate.

13. The system of claim 1, wherein the minimum flow rate (MinFR) comprises a first target flow rate less than about 50 cc/minute and a second target flow rate less than about 100 cc/minute.

14. The system of claim 1, wherein the pump (112) is coupled to a motor (114) for driving the pump (112) in response to the application of power from a power source, and the controller (170) is configured further to increase negative pressure at the wound site when the wound pressure (WP) is less than a minimum wound pressure (WPMin) by increasing power applied to the motor (114).

15. The system of claim 17, wherein the pump (112) is coupled to a motor (114) for driving the pump (112) in response to the application of power from a power source, and the controller (170) is configured further to decrease negative-pressure source at the wound site when the wound pressure (WP) is greater than a maximum wound pressure (WPMax) by decreasing the power applied to the motor (114).

## Patentansprüche

1. Ein System zum Fördern einer Heilung einer Wunde an einer Gewebestelle, aufweisend:
einen Verband (115), aufweisend ein poröses Polster (120), das konfiguriert ist, um an der Gewebestelle positioniert zu werden, und das angepasst ist, um durch eine Abdeckung (125) bedeckt zu werden, um eine therapeutische Umgebung auszubilden, die von der äußeren Umgebung getrennt ist, zum Aufrechterhalten eines Wundendrucks (WP);
eine Unterdruckquelle (110), aufweisend eine Pumpe (112), die angepasst ist, um einen Pumpendruck (PP) zu erzeugen und die ferner angepasst ist, um mit dem porösen Polster (120) fluidisch gekoppelt zu sein, zum Anlegen von Unterdruck an die Gewebestelle;
einen Fließgeschwindigkeitssensor, der einen Eingang, der zwischen der Pumpe (112) und dem porösen Polster (120) fluidisch gekoppelt ist, und einen Ausgang zum Bereitstellen eines Fließgeschwindigkeitssignals, das eine Fließgeschwindigkeit (FR) von Fluiden darstellt, die eine Leckage zwischen der Pumpe (112) und dem porösen Polster (120) anzeigt, aufweist;
eine Steuerung (170), die einen Eingang, der mit dem Ausgang des Fließgeschwindigkeitssensors gekoppelt ist, und einen Ausgang aufweist, wobei die Steuerung (170) konfiguriert ist, um (i) eine Fließgeschwindigkeit (FR) basierend auf dem Fließgeschwindigkeitssignal zu bestimmen, (ii) die Fließgeschwindigkeit (FR) mit einer minimalen Fließgeschwindigkeit (MinFR) zu vergleichen und (iii) ein Signal einer geringen Leckage an dem Ausgang der Steuerung (170) zu erzeugen, wenn die Fließgeschwindigkeit (FR) kleiner als die minimale Fließgeschwindigkeit (MinFR) ist; und
einen Regler (165), der mit dem Ausgang der Steuerung (170) gekoppelt ist und angepasst ist, um die Fließgeschwindigkeit (FR) durch Erhöhen der Leckage in die therapeutische Umgebung als Reaktion auf das Auftreten eines Signals geringer Leckage zu erhöhen.

2. Das System nach Anspruch 1, wobei der Fließgeschwindigkeitssensor einen ersten Drucksensor (156), der einen ersten Eingang zum Erfassen des Pumpendrucks (PP) und einen ersten Ausgang zum Bereitstellen eines Signals, das den Pumpendruck (PP) anzeigt, aufweist, und einen zweiten Drucksensor (155) aufweist, der einen zweiten Eingang zum Erfassen des Wundendrucks (WP) und einen zweiten Ausgang zum Bereitstellen eines Signals, das den Wundendruck (WP) anzeigt, aufweist, und wobei die Steuerung (170) mit dem ersten Ausgang des ersten Drucksensors (156) und dem zweiten Ausgang des zweiten Drucksensors (155) elektrisch gekoppelt ist und ferner konfiguriert ist, um die Fließgeschwindigkeit (FR) basierend auf der Differenz zwischen dem Pumpendruck (PP) und dem Wundendruck (WP) zu bestimmen.

3. Das System nach Anspruch 1, wobei der Regler (165) konfiguriert ist, um die Leckage durch Ablassen von Gasen aus der äußeren Umgebung in die therapeutische Umgebung zu erhöhen.

4. Das System nach Anspruch 3, wobei der Regler (165) ein Steuerventil ist.

5. Das System nach Anspruch 3, wobei die Leckage einen ersten Leckageschwellenwert und einen zweiten Leckageschwellenwert aufweist.

6. Das System nach Anspruch 3, wobei der Regler (165) ferner konfiguriert ist, um die Leckage von null auf einen ersten Leckageschwellenwert zu erhöhen.

7. Das System nach Anspruch 6, wobei der Regler (165) ferner konfiguriert ist, um die Leckage durch Ablassen von Gasen bei einer festen Geschwindigkeit zu erhöhen.

8. Das System nach Anspruch 6, wobei der Regler (165) ferner konfiguriert ist, um die Leckage durch Ablassen von Gasen bei einer veränderbaren Geschwindigkeit zu erhöhen.

9. Das System nach Anspruch 8, wobei der Regler (165) ferner konfiguriert ist, um die Amplitude, eine Frequenz oder einen Arbeitszyklus der veränderbaren Geschwindigkeit zu verändern.

10. Das System nach Anspruch 1, wobei die minimale Fließgeschwindigkeit (MinFR) kleiner als etwa 50 cc/Minute oder kleiner als etwa 100 cc/Minute ist.

11. Das System nach Anspruch 1, wobei die minimale Fließgeschwindigkeit (MinFR) zwei oder mehr Zielfließgeschwindigkeiten aufweist.

12. Das System nach Anspruch 14, wobei die Steuerung (170) ferner konfiguriert ist, um die Fließgeschwindigkeit (FR) basierend auf einer ersten Zielfließgeschwindigkeit und einer zweiten Zielfließgeschwindigkeit zu justieren.

13. Das System nach Anspruch 1, wobei die minimale Fließgeschwindigkeit (MinFR) eine erste Zielfließgeschwindigkeit, die kleiner als etwa 50 cc/Minute ist, und eine zweite Zielfließgeschwindigkeit, die kleiner als etwa 100 cc/Minute ist, aufweist.

14. Das System nach Anspruch 1, wobei die Pumpe (112) mit einem Motor (114) zum Antreiben der Pumpe (112) als Reaktion auf das Anlegen von Leistung aus einer Leistungsquelle gekoppelt ist und die Steuerung (170) ferner konfiguriert ist, um den Unterdruck an der Wundstelle, wenn der Wundendruck (WP) kleiner als ein minimaler Wundendruck (WPMin) ist, durch Erhöhen der an den Motor (114) angelegten Leistung zu erhöhen.

15. Das System nach Anspruch 17, wobei die Pumpe (112) mit einem Motor (114) zum Antreiben der Pumpe (112) als Reaktion auf das Anlegen von Leistung von einer Leistungsquelle gekoppelt ist und die Steuerung (170) ferner konfiguriert ist, um die Unterdruckquelle an der Wundstelle, wenn der Wundendruck (WP) größer als ein maximaler Wundendruck (WPMax) ist, durch Verringern der an den Motor (114) angelegten Leistung zu verringern.

## Revendications

1. Système favorisant la cicatrisation d'une plaie au niveau d'un site tissulaire, comprenant :
un pansement (115) comportant un tampon poreux (120) conçu pour être positionné au niveau du site tissulaire et adapté pour être recouvert par un champ (125) afin de former un environnement thérapeutique séparé de l'environnement externe pour le maintien d'une pression de plaie (WP) ;
une source de pression négative (110) comportant une pompe (112) adaptée pour générer une pression de pompe (PP) et adaptée en outre pour être accouplée fluidiquement au tampon poreux (120) afin d'appliquer une pression négative au site tissulaire ;
un capteur de débit ayant une entrée accouplée fluidiquement entre la pompe (112) et le tampon poreux (120) et une sortie pour fournir un signal de débit représentant un débit (FR) de fluides indiquant une fuite entre la pompe (112) et le tampon poreux (120) ;
un dispositif de commande (170) ayant une entrée couplée à la sortie du capteur de débit et une sortie, dans lequel le dispositif de commande (170) est configuré pour (i) déterminer un débit (FR) en fonction du signal de débit, (ii) comparer le débit (FR) à un débit minimal (MinFR), et pour (iii) générer un signal de faible fuite à la sortie du dispositif de commande (170) lorsque le débit (FR) est inférieur au débit minimal (MinFR) ; et
un régulateur (165) couplé à la sortie du dispositif de commande (170) et adapté pour augmenter le débit (FR) en augmentant la fuite dans l'environnement thérapeutique en réponse à l'apparition d'un signal de faible fuite.

2. Système selon la revendication 1, dans lequel le capteur de débit comprend un premier capteur de pression (156) ayant une première entrée pour détecter la pression de pompe (PP) et une première sortie pour fournir un signal indiquant la pression de pompe (PP), et un second capteur de pression (155) ayant une seconde entrée pour détecter la pression de plaie (WP) et une seconde sortie pour fournir un signal indiquant la pression de plaie (WP), et dans lequel le dispositif de commande (170) est couplé électriquement à la première sortie du premier capteur de pression (156) et à la seconde sortie du second capteur de pression (155), et est en outre configuré pour déterminer le débit (FR) en fonction de la différence entre la pression de pompe (PP) et la pression de plaie (WP).

3. Système selon la revendication 1, dans lequel le régulateur (165) est configuré pour augmenter la fuite en évacuant des gaz de l'environnement extérieur vers l'environnement thérapeutique.

4. Système selon la revendication 3, dans lequel le régulateur (165) est une vanne de régulation.

5. Système selon la revendication 3, dans lequel la fuite présente un premier seuil de fuite et un second seuil de fuite.

6. Système selon la revendication 3, dans lequel le régulateur (165) est en outre configuré pour augmenter la fuite de zéro à un premier seuil de fuite.

7. Système selon la revendication 6, dans lequel le régulateur (165) est en outre configuré pour augmenter la fuite en évacuant des gaz à un débit fixe.

8. Système selon la revendication 6, dans lequel le régulateur (165) est en outre configuré pour augmenter la fuite en évacuant des gaz à un débit variable.

9. Système selon la revendication 8, dans lequel le régulateur (165) est en outre configuré pour faire varier l'amplitude, la fréquence ou le rapport cyclique du débit variable.

10. Système selon la revendication 1, dans lequel le débit minimal (MinFR) est inférieur à environ 50 cm³/minute ou inférieur à environ 100 cm³/minute.

11. Système selon la revendication 1, dans lequel le débit minimal (MinFR) comprend deux débits cibles ou plus.

12. Système selon la revendication 14, dans lequel le dispositif de commande (170) est en outre configuré pour ajuster le débit (FR) en fonction d'un premier débit cible et d'un second débit cible.

13. Système selon la revendication 1, dans lequel le débit minimal (MinFR) comprend un premier débit cible inférieur à environ 50 cm³/minute et un second débit cible inférieur à environ 100 cm³/minute.

14. Système selon la revendication 1, dans lequel la pompe (112) est couplée à un moteur (114) pour entraîner la pompe (112) en réponse à l'application de puissance à partir d'une source de puissance, et le dispositif de commande (170) est en outre configuré pour augmenter une pression négative au niveau du site de plaie lorsque la pression de plaie (WP) est inférieure à une pression minimale de plaie (WPMin), en augmentant la puissance appliquée au moteur (114).

15. Système selon la revendication 17, dans lequel la pompe (112) est couplée à un moteur (114) pour entraîner la pompe (112) en réponse à l'application de puissance à partir d'une source de puissance, et le dispositif de commande (170) est en outre configuré pour diminuer la source de pression négative au niveau du site de plaie lorsque la pression de plaie (WP) est supérieure à une pression maximale de plaie (WPMax), en diminuant la puissance appliquée au moteur (114).
